# EUROPÄISCHE PATENTSCHRIFT

(11) **EP 0 437 177 B1**
(45) Veröffentlichungstag und Bekanntmachung des Hinweises auf die Patenterteilung: **20.07.1994**
(21) Anmeldenummer: 90810932.5
(22) Anmeldetag: 29.11.1990
(51) Int. Cl.: C07C 323/67, C07C 319/14, C07C 311/16, C07F 7/10

(54) **Verfahren zur Herstellung von Phenylsulfonamiden**
Process for the preparation of phenylsulfonamides
Procédé pour la préparation de phénylsulfonamides

(30) Priorität: 08.12.1989 CH 4413/89
(43) Veröffentlichungstag der Anmeldung: 17.07.1991
(73) Patentinhaber: CIBA-GEIGY AG, 4002 Basel (CH)
(72) Erfinder: Wirz, Bernard, CH-4127 Birsfelden (CH); Willy, Meyer, CH-4125 Riehen (CH); Stutz, Wolfgang, Dr., CH-4333 Münchwilen (CH)

(56) Entgegenhaltungen:
- EP-A- 0 074 282
- DD-A- 251 342
- DE-A- 1 493 664
- DE-A- 2 002 065
- US-A- 4 141 916
- JOURNAL OF ORGANIC CHEMISTRY OF THE USSR, Band 7, Nr. 1, 1971, Seiten 114-116, New York, US; M.M. KREMLEV et al.: "Investigations into the arenesulfonamides. LV. Arenesulfoamidation of triphenylmethane"
- CHEMICAL ABSTRACTS, Band 76, 1972, Seite 456, Zusammenfassung Nr. 126521h, Columbus, Ohio, US; M.M. KREMLEV et al.: "Arenesulfonamides. XXIII. Benzenesulfonamidation of diphenyl- and triphenylmethanes"

## Beschreibung

Die vorliegende Erfindung betrifft ein Verfahren zur Herstellung von 2-(2-Halogenethylthio)-phenylsulfonamiden der Formel I 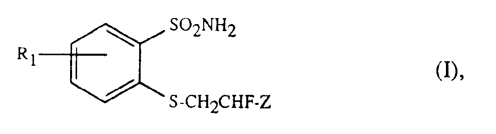
worin
R₁ Wasserstoff, C₁-C₅-Alkyl oder C₂-C₅-Alkenyl; und
Z Wasserstoff, Fluor oder Chlor bedeutet.

Die 2-(2-Halogenethylthio)-phenylsulfonamide der Formel I sind wertvolle Zwischenprodukte zur Herstellung von herbizid wirksamen N-Phenylsulfonyl-N′-pyrimidinyl- und -triazinylharnstoffen wie sie beispielsweise in der europäischen Patentanmeldung Nr. 44808 offenbart sind.

Solche von den 2-(2-Halogenethylthio)-phenylsulfonamiden der Formel I abgeleiteten Phenylsulfonylharnstoffe zeichnen sich durch ein günstiges Abbauverhalten aus. Es besteht somit ein Bedarf nach einem vorteilhaften Herstellungsverfahren für die 2-(2-Halogenethylthio)-phenylsulfonamide der Formel I.

Aus EP-A-0074282 ist bekannt, 2-alkylthiosubstituierte Phenylsulfonamide in der Weise herzustellen, indem man
1) N-tert.-Butylphenylsulfonamid mit 2 Äquivalenten n-Butyllithium in das Dilithiumsalz der Formel VIII 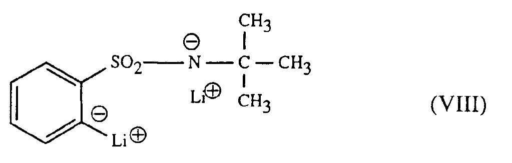 überführt, dieses mit
2) Schwefel und
3) einem Alkylhalogenid umsetzt und anschliessend
4) die Schutzgruppe abspaltet.

Dieses Verfahren besitzt jedoch den schwerwiegenden Nachteil, dass für die Umsetzung pro eingesetztes Äquivalent des Phenylsulfonamids 2 Äquivalente des kostspieligen n-Butyllithiums benötigt werden.

Es ist daher das Ziel der vorliegenden Erfindung, ein Verfahren bereitzustellen, welches die Herstellung von 2-(2-Halogenethylthio)-phenylsulfonamiden ausgehend von leicht zugänglichen Ausgangsstoffen auf besonders kostengünstige und einfache Weise in guten Ausbeuten ermöglicht.

Es wurde nun gefunden, dass man die 2-(2-Halogenethylthio)-phenylsulfonamide der Formel I in vorteilhafter Weise herstellen kann, wenn man
A) ein Phenylsulfonamid der Formel II 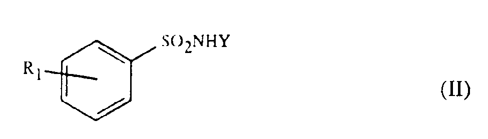 worin
   R₁ die unter Formel I angegebene Bedeutung hat und
   Y für eine tertiäre Alkylgruppe mit 4 bis 8 Kohlenstoffatomen, Benzyl, Diphenylmethyl, Triphenylmethyl oder eine Silylgruppe der Formel VII

   -Si(R₂R₃R₄) (VII),

   worin R₂, R₃ und R₄ unabhängig voneinander C₁-C₆-Alkyl oder Phenyl bedeuten, steht, nacheinander ohne Isolierung von Zwischenprodukten mit
   a) einer starken Base der Formel IX

      MeB (IX),

      worin Me für Natrium oder Kalium, B für Wasserstoff, OH, NH₂ oder OR₅ und R₅ für C₁-C₅-Alkyl steht, in die Verbindung der Formel III 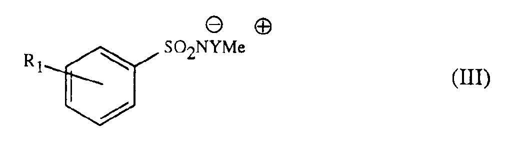 worin Me, R₁ und Y die oben angegebene Bedeutung haben und R₁ die unter Formel I angegebene Bedeutung hat,
      überführt, diese mit
   b) einem Äquivalent n-Butyllithium und
   c) Schwefel
      in die Verbindung der Formel IV 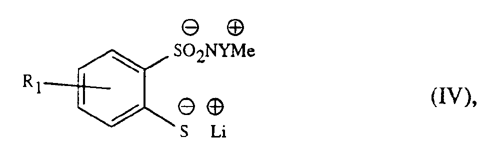 worin Me, R₁ und Y die oben angegebene Bedeutung haben und R₁ die unter Formel I angegebene Bedeutung hat,
      überführt, diese mit
   d) einem 2-Halogenfluorethan der Formel V

      X-CH₂CHF-Z (V)

      worin X für Chlor oder Brom steht und Z die unter Formel I angegebene Bedeutung hat, zu einem Phenylsulfonamid der Formel VI 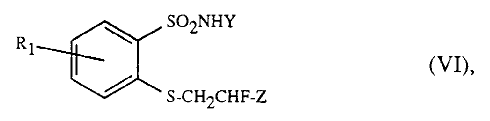 worin Z und R₁ die unter Formel I angegebene Bedeutung und Y die unter Formel II angegebene Bedeutung haben, umsetzt und
B) anschliessend die Gruppe Y abspaltet.

Ausgangs- und Endprodukte des erfindungsgemässen Verfahrens sind bekannt. Verbindungen der Formel V und IX sind ebenfalls bekannt und im Handel erhältlich.

Das Phenylsulfonamid der Formel VI sowie die Salze der Formel III sind neu und bilden ebenfalls einen Gegenstand der vorliegenden Erfindung.

Die in den Definitionen der Substituenten vorkommenden Alkylgruppen können geradkettig oder verzweigt sein und stehen beispielsweise für Methyl, Ethyl, n-Propyl, iso-Propyl, n-Butyl, sek.-Butyl, iso-Butyl, tert.-Butyl, n-Pentyl oder die isomeren Pentyl, n-Hexyl oder die isomeren Hexyl. Vorzugsweise besitzen die Alkylgruppen 1 bis 4 Kohlenstoffatome.

Die in den Definitionen des Substituenten Y vorkommenden tertiären Alkylgruppen mit 4 bis 8 Kohlenstoffatomen stehen beispielsweise für tert.-Butyl, 3-Methyl-pent-3-yl, 2-Methyl-but-2-yl, 3-Ethyl-pent-3-yl oder 4-Methyl-hept-4-yl, besonders bevorzugt steht Y für tert.-Butyl.

Die im Substituenten R₁ vorkommenden C₂-C₅-Alkenylreste können geradkettig oder verzweigt sein. Bevorzugt sind Alkenylreste mit einer Kettenlänge von zwei bis drei Kohlenstoffatomen. Beispiele für C₂-C₅-Alkenylreste sind: Vinyl, Allyl, Methallyl, 1-Methylvinyl, 2-Methylvinyl, But-2-en-1-yl oder Pent-3-en-1-yl. Bevorzugt ist Vinyl und Allyl.

Für die Umsetzung gemäss Verfahrensschritt Aa) geeignete Lösungsmittel sind offenkettige, verzweigte, unverzweigte oder zyklische Alkane sowie Aromate. Bevorzugte Lösungsmittel sind Toluol, Xylol, Mesitylen sowie Alkane mit 6 bis 8 Kohlenstoffatomen wie n-Hexan, Cyclohexan oder n-Oktan. Werden für den Verfahrensschritt Aa) Verbindungen der Formel IX verwendet, in denen B für OH oder OR₅ steht, können als Lösungsmittel auch primäre, sekundäre oder tertiäre Alkohole mit 1 bis 5 Kohlenstoffatomen eingesetzt werden, wie beispielsweise Methanol, Ethanol, Iso-Propanol oder Butanol. Bevorzugte Alkohole sind dabei Methanol und Ethanol.

Bevorzugte starke Basen der Formel IX sind die Hydroxide und Alkoholate. Besonders bevorzugt ist Natriumhydroxid, Kaliumhydroxid, Natriummethylat, Natriumethylat sowie Kaliummethylat.

Wenn Y für eine Silylgruppe der Formel VII steht, erfolgt die Umsetzung zweckmässigerweise unter aprotischen Bedingungen. Bevorzugte Basen der Formel IX sind hier die Amide und die Hydride; besonders bevorzugte Basen sind Natriumamid, Natriumhydrid und Kaliumhydrid.

Für die Verfahrensschritte Ab) bis Ad) geeignete Lösungsmittel sind beispielsweise Verbindungen oder Gemische aus der Gruppe der offenkettigen und zyklischen Ether wie Dioxan, Tetrahydrofuran, Diethylether, Diisopropylether, Dimethoxymethan und 1,2-Dimethoxyethan, aromatische Kohlenwasserstoffe wie Benzol, Toluol oder Xylol, sowie offenkettige Alkane wie n-Pentan oder n-Hexan. Ein bevorzugtes Lösungsmittel ist Tetrahydrofuran.

Die Umsetzung des 2-Halogenfluorethans der Formel V gemäss Verfahrensschritt Ad) kann sowohl bei Normaldruck als auch bei erhöhtem Druck erfolgen. Als gut geeignet hat sich ein Druckbereich von 1 bis 10 bar erwiesen, wobei ein Bereich von 1 bis 3 bar besonders bevorzugt ist.

Für die Abspaltung der Gruppe Y gemäss Verfahrensschritt B) geeignete Lösungsmittel sind beispielsweise cyclische und offenkettige Ether wie die für die Verfahrensschritte Ab) bis Ad) genannten sowie aliphatische Ketone wie Aceton, Methylethylketon, Diethylketon, Methylisopropylketon oder Methylisobutylketon.
Für diesen Verfahrensschritt besonders gut geeignete Lösungsmittel sind Dioxan sowie Methylisobutylketon.

Die Abspaltung der Gruppe Y erfolgt in Gegenwart einer Säure. Besonders vorteilhaft verläuft die Reaktion mit Trifluoressigsäure oder Salzsäure, wobei Salzsäure ganz besonders bevorzugt ist.

Das erfindungsgemässe Verfahren kann in einem weiten Temperaturbereich durchgeführt werden. Mit Vorteil setzt man beim Verfahrensschritt A) das Phenylsulfonamid der Formel II nacheinander ohne Isolierung von Zwischenprodukten mit
a) einer starken Base der Formel IX bei einer Temperatur von +20 bis +250°C, vorzugsweise von +60 bis +150°C zur Verbindung der Formel III um, welche
b) mit n-Butyllithium bei einer Temperatur von -80 bis +50°C, vorzugsweise-20 bis +30°C; und
c) mit Schwefel bei einer Temperatur von -50 bis +50°C, vorzugsweise-10 bis +30°C, in die Verbindung der Formel IV überführt wird, die anschliessend unmittelbar
d) mit einem 2-Halogenfluorethan der Formel V bei einer Temperatur von 0 bis +80°C, vorzugsweise +20 bis +60°C und einen Druck von 1 bis 3 bar, zur Verbindung der Formel VI umgesetzt wird.

Für das hydrolytische Abspalten der Gruppe Y gemäss Verfahrensschritt B) hat sich ein Temperaturbereich von -20°C bis +60°C, vorzugsweise -20 bis +30°C, als geeignet erwiesen.

Besonders vorteilhaft lassen sich diejenigen Verbindungen der Formel I herstellen, in denen R₁ für Wasserstoff steht wobei Z vorzugsweise Wasserstoff bedeutet.

In einer besonders bevorzugten Variante des erfindungsgemässen Verfahrens wird das 2-(2-Halogenethylthio)-phenylsulfonamid der Formel I hergestellt, indem man
A) N-tert.-Butyl-phenylsulfonamid nacheinander ohne Isolierung von Zwischenprodukten mit
   a) Natriummethylat oder Kaliumhydroxid bei einer Temperatur von +60 bis +150°C in die Verbindung der Formel IIIa 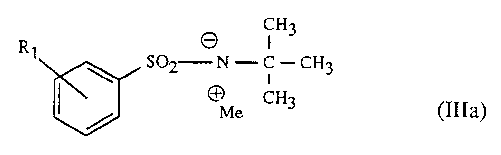 worin Me für Natrium oder Kalium steht und R₁ die unter Formel I angegebene Bedeutung hat, überführt, diese
   b) mit einem Äquivalent n-Butyllithium bei einer Temperatur von -20 bis +30°C und
   c) Schwefel bei einer Temperatur von -20 bis +30°C
      in die Verbindung der Formel IVa 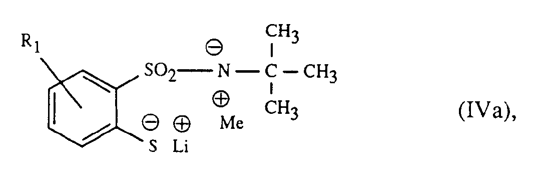 worin Me für Natrium oder Kalium steht und R₁ die unter Formel I angegebene Bedeutung hat, überführt, diese mit
   d) einem 2-Halogenfluorethan der Formel V bei einer Temperatur von +20 bis +60°C und einem Druck von 1 bis 3 bar zu einem 2-(2-Halogenethylthio)-N-tert.-butyl-phenylsulfonamid der Formel VIa 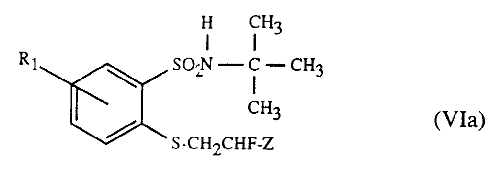 worin Z und R₁ die unter Formel I angegebene Bedeutung haben, umsetzt, und
B) anschliessend die tert.-Butylgruppe bei einer Temperatur von -20 bis +30°C in Gegenwart von Salzsäure abspaltet. In dieser bevorzugten Variante des erfindungsgemässen Verfahrens stehen Z und R₁ vorzugsweise für Wasserstoff.

Das erfindungsgemässe Verfahren zeichnet sich durch zahlreiche vorteilhafte Eigenschaften aus. Die Reaktionsführung ist unkompliziert. Die nur zweistufige Reaktion ist ökonomisch besonders vorteilhaft und erfordert nur geringen apparativen Aufwand. Ausserdem weist das Verfahren eine hohe Ausbeute und gute Produktqualität auf.

Anstelle von 2 Äquivalenten des kostspieligen n-Butyllithiums pro eingesetztes Äquivalent Phenylsulfonamid wird für das erfindungsgemässe Verfahren nur ein Äquivalent n-Butyllithium sowie wesentlich kostengünstigere Natrium- oder Kaliumbasen benötigt.

Das erfindungsgemässe Verfahren wird durch die folgenden Beispiele näher erläutert.

### Herstellungsbeispiele:

### Beispiel 1: Herstellung von 2-(2-Fluorethylthio)-N-tert.-butyl-phenylsulfonamid

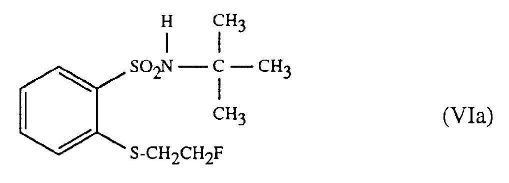

Zu einer Suspension von 9,4 g (0,15 Mol) pulverförmigem Kaliumhydroxid (90%ig) in 230 ml Toluol lässt man am Wasserabscheider unter Rückfluss bei einem Druck von 0,4 bar 32 g (0,15 Mol) N-(tert.-Butyl)-phenylsulfonamid in 120 ml Toluol zutropfen. Anschliessend wird das entstandene Wasser azeotrop abdestilliert und das Toluol unter reduziertem Druck entfernt. Aus dem erhaltenen Rückstand kann gewünschtenfalls das Kaliumsalz der Formel III zur Charakterisierung isoliert werden. Es wurden folgende spektroskopische Daten erhalten:

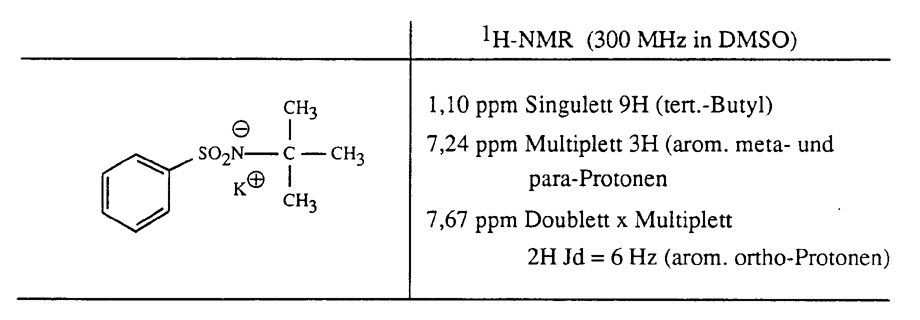

Der Rückstand wird in einer Stickstoffatmosphäre mit 120 ml Tetrahydrofuran aufgeschlämmt und anschliessend auf -15°C gekühlt. Zu dieser Suspension lässt man innerhalb von 30 Minuten 100 ml (0,16 Mol) einer 1,6 molaren n-Butyllithiumlösung in n-Hexan zutropfen. Nach 4-stündigem Rühren bei Raumtemperatur und erneutem Abkühlen auf 0°C gibt man 5,6 g (0,17 Mol) Schwefel hinzu und rührt die Reaktionsmischung anchliessend für weitere 3 Stunden bei Raumtemperatur. Zum erhaltenen Reaktionsgemisch werden 21,6 g (0,17 Mol) 1-Brom-2-fluorethan zugegeben und die Mischung anschliessend 18 Stunden lang bei Raumtemperatur gerührt. Anschliessend wird die Reaktionsmischung mit 100 ml Toluol verdünnt und auf ein Gemisch aus 100 ml Eis und 30 ml konzentrierter Salzsäure gegeben. Die organische Phase wird anschliessend abgetrennt und am Vakuum bis zur Gewichtskonstanz eingeengt. Man erhält 43,5 g 2-(2-Fluorethylthio)-N-tert.-butyl-phenylsulfonamid (VIa, 83%ig d. Th.) in Form eines braunen Öls mit einer Reinheit von 83%.

### Beispiel 2: Herstellung von 2-(2-Fluorethylthio)-phenylsulfonamid

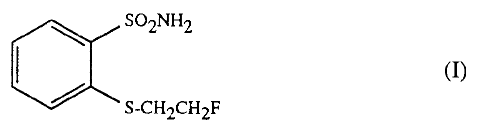

Zu einer Lösung aus 3,88 g gemäss Beispiel H1 erhaltenen 2-(2-Fluorethylthio)-N-tert.-butyl-phenylsulfonamids in 25 ml Trichlormethan gibt man bei einer Temperatur von 0°C 25 ml Trifluoressigsäure hinzu. Nach 18-stündigem Rühren bei einer Temperatur von +25°C wird das erhaltene braune Reaktionsgemisch lyophilisiert. Nach chromatographischer Reinigung des öligen Rückstandes mit 1) Essigsäureethylester/Petrolether (1:1) über Kieselgel und 2) Dichlormethan/Aceton (9:1) über Kieselgel werden die erhaltenen Fraktionen eingedampft. Man erhält 1,5 g (60,5% der Theorie) kristallines 2-(2-Fluorethylthio)-phenylsulfonamid.

### Beispiel 3: Herstellung von 2-(2-Fluorethylthio)-phenylsulfonamid

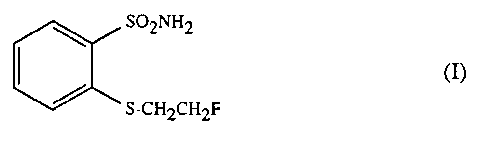

Zu einer Lösung von 18,2 g gemäss Beispiel H1 erhaltenen 2-(2-Fluorethylthio)-N-tert.-butyl-phenylsulfonamids (80%ig, 0,05 Mol) in 70 ml Methyl-isobutylketon gibt man bei einer Temperatur von +20 bis +25°C unter Kühlung 70 ml konzentrierte Salzsäure tropfenweise hinzu. Nach Halten des Reaktionsgemisches über 40 Stunden bei Raumtemperatur wird mit 30%iger Natronlauge neutralisiert und anschliessend die wässrige Phase abgetrennt. Nach dem Entfernen des Lösungsmittels unter reduziertem Druck erhält man aus der organischen Phase 15,8 g eines kristallinen Rückstandes, der das 2-(2-Fluorethylthio)-phenylsulfonamid der Formel I mit einem Gehalt von 70% und in einer Ausbeute von 94% d. Th. enthält. Nach Umkristallisation aus Isopropanol erhält man 10,1 g (79% d. Th.) 2-(2-Fluorethylthio)-phenylsulfonamid der Formel I mit einem Gehalt von 92%.

## Patentansprüche (Patentansprüche für folgende(n) Vertragsstaat(en): AT, BE, CH, DE, DK, FR, GB, GR, IT, LI, LU, NL, SE)

1. Verfahren zur Herstellung von 2-(2-Halogenethylthio)-phenylsulfonamiden der Formel I 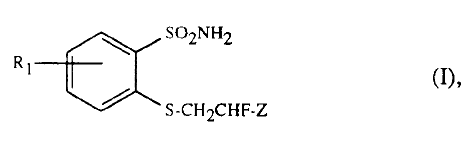 worin
R₁ Wasserstoff, C₁-C₅-Alkyl oder C₂-C₅-Alkenyl; und
Z Wasserstoff, Fluor oder Chlor bedeutet;
dadurch gekennzeichnet, dass man
A) ein Phenylsulfonamid der Formel II 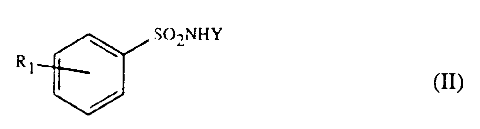 worin
R₁ die unter Formel I angegebene Bedeutung hat und
Y für eine tertiäre Alkylgruppe mit 4 bis 8 Kohlenstoffatomen, Benzyl, Diphenylmethyl, Triphenylmethyl oder eine Silylgruppe der Formel VII
-Si(R₂R₃R₄) (VII),
worin R₂, R₃ und R₄ unabhängig voneinander C₁-C₆-Alkyl oder Phenyl bedeuten, steht, nacheinander ohne Isolierung von Zwischenprodukten mit
a) einer starken Base der Formel IX
MeB (IX),
worin Me für Natrium oder Kalium, B für Wasserstoff, OH, NH₂ oder OR₅ und R₅ für C₁-C₅-Alkyl steht, in die Verbindung der Formel III 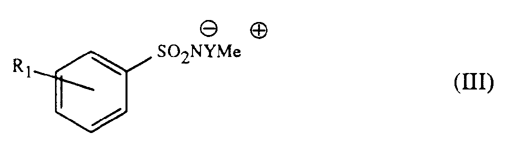 worin Me, R₁ und Y die oben angegebene Bedeutung haben,
überführt, diese mit
b) einem Äquivalent n-Butyllithium und
c) Schwefel
in die Verbindung der Formel IV 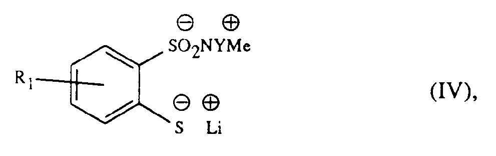 worin Me, R₁ und Y die oben angegebene Bedeutung haben,
überführt, diese mit
d) einem 2-Halogenfluorethan der Formel V
X-CH₂CHF-Z (V)
worin X für Chlor oder Brom steht und Z die unter Formel I angegebene Bedeutung hat,
zu einem Phenylsulfonamid der Formel VI 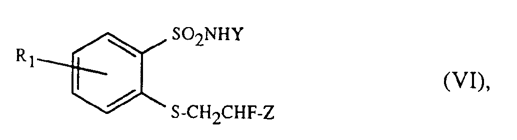 worin Z und R₁ die unter Formel I angegebene Bedeutung und Y die unter Formel II angegebene Bedeutung haben, umsetzt und
B) anschliessend die Gruppe Y abspaltet.

2. Verfahren nach Anspruch 1, dadurch gekennzeichnet, dass R₁ für Wasserstoff steht.

3. Verfahren nach Anspruch 1, dadurch gekennzeichnet, dass Z für Wasserstoff steht.

4. Verfahren nach Anspruch 1, dadurch gekennzeichnet, dass B für OH oder OR₅ steht.

5. Verfahren nach Anspruch 1, dadurch gekennzeichnet, dass man als starke Base der Formel IX Kaliumhydroxid oder Natriummethylat verwendet.

6. Verfahren nach Anspruch 1, dadurch gekennzeichnet, dass X für Brom steht.

7. Verfahren nach Anspruch 1, dadurch gekennzeichnet, dass Y für tert.-Butyl steht.

8. Verfahren nach Anspruch 1, dadurch gekennzeichnet, dass man für die Abspaltung der Gruppe Y Salzsäure verwendet.

9. Verfahren nach Anspruch 1, dadurch gekennzeichnet, dass man
A) N-tert.-Butyl-phenylsulfonamid nacheinander ohne Isolierung von Zwischenprodukten mit
a) Natriummethylat oder Kaliumhydroxid bei einer Temperatur von +60 bis +150°C in die Verbindung der Formel IIIa 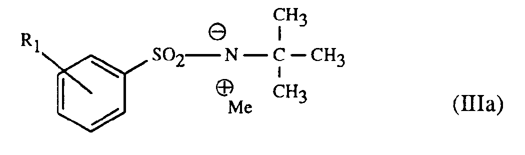 worin Me für Natrium oder Kalium steht und R₁ die unter Formel I in Anspruch 1 angegebene Bedeutung hat, überführt, diese
b) mit einem Äquivalent n-Butyllithium bei einer Temperatur von -20 bis +30°C und
c) Schwefel bei einer Temperatur von -20 bis +30°C
in die Verbindung der Formel IVa 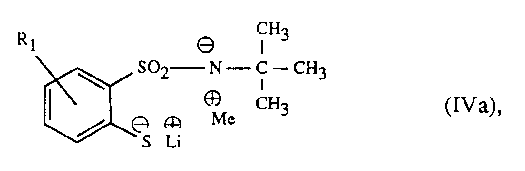 worin Me für Natrium oder Kalium steht und R₁ die unter Formel I in Anspruch 1 angegebene Bedeutung hat, überführt, diese mit
d) einem 2-Halogenfluorethan der Formel V bei einer Temperatur von +20 bis +60°C und einem Druck von 1 bis 3 bar
zu einem 2-(2-Halogenethylthio)-N-tert.-butyl-phenylsulfonamid der Formel VIa 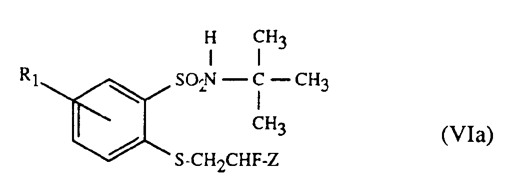 worin Z und R₁ die unter Formel I angegebene Bedeutung haben,
umsetzt, und
B) anschliessend die tert.-Butylgruppe bei einer Temperatur von -20 bis +30°C in Gegenwart von Salzsäure abspaltet.

10. Verfahren nach Anspruch 9, dadurch gekennzeichnet, dass R₁ für Wasserstoff steht.

11. Verfahren nach Anspruch 9, dadurch gekennzeichnet, dass Z für Wasserstoff steht.

12. Verbindungen der Formel VI 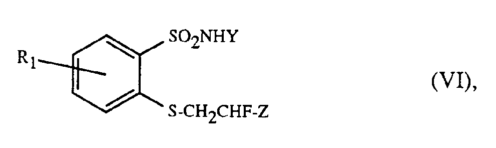 worin R₁ die unter Formel I in Anspruch 1 angegebene Bedeutung hat und
Y für eine tertiäre Alkylgruppe mit 4 bis 8 Kohlenstoffatomen, Benzyl, Diphenylmethyl, Triphenylmethyl oder eine Silylgruppe der Formel VII
-Si(R₂R₃R₄) (VII),
worin R₂, R₃ und R₄ unabhängig voneinander C₁-C₆-Alkyl oder Phenyl bedeuten, steht.

13. Verbindungen der Formel VI gemäss Anspruch 12, dadurch gekennzeichnet, dass R₁ für Wasserstoff steht.

14. Verbindungen der Formel VI gemäss Anspruch 12, dadurch gekennzeichnet, dass Z für Wasserstoff steht.

15. Verbindungen der Formel VIa 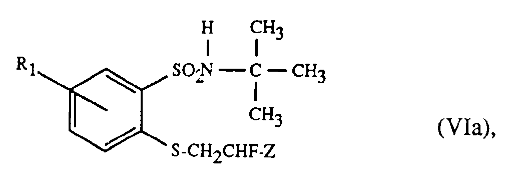 worin R₁ und Z die unter Formel I in Anspruch 1 angegebene Bedeutung haben.

16. Verbindungen der Formel VIa gemäss Anspruch 15, dadurch gekennzeichnet, dass R₁ für Wasserstoff steht.

17. Verbindungen der Formel VIa gemäss Anspruch 15, dadurch gekennzeichnet, dass Z für Wasserstoff steht.

## Patentansprüche (Patentansprüche für folgende(n) Vertragsstaat(en): ES)

1. Verfahren zur Herstellung von 2-(2-Halogenethylthio)-phenylsulfonamiden der Formel I 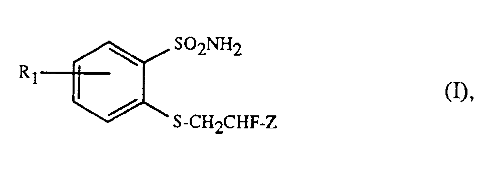 worin
R₁ Wasserstoff, C₁-C₅-Alkyl oder C₂-C₅-Alkenyl; und
Z Wasserstoff, Fluor oder Chlor bedeutet;
dadurch gekennzeichnet, dass man
A) ein Phenylsulfonamid der Formel II 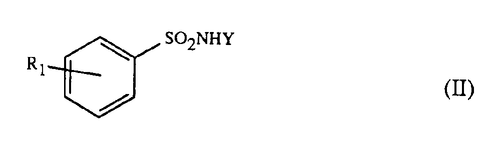 worin
R₁ die unter Formel I angegebene Bedeutung hat und
Y für eine tertiäre Alkylgruppe mit 4 bis 8 Kohlenstoffatomen, Benzyl, Diphenylmethyl, Triphenylmethyl oder eine Silylgruppe der Formel VII
-Si(R₂R₃R₄) (VII),
worin R₂, R₃ und R₄ unabhängig voneinander C₁-C₆-Alkyl oder Phenyl bedeuten, steht, nacheinander ohne Isolierung von Zwischenprodukten mit
a) einer starken Base der Formel IX
MeB (IX),
worin Me für Natrium oder Kalium, B für Wasserstoff, OH, NH₂ oder OR₅ und R₅ für C₁-C₅-Alkyl steht, in die Verbindung der Formel III 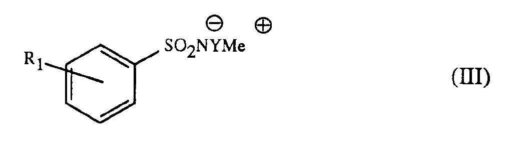 worin Me, R₁ und Y die oben angegebene Bedeutung haben,
überführt, diese mit
b) einem Äquivalent n-Butyllithium und
c) Schwefel
in die Verbindung der Formel IV 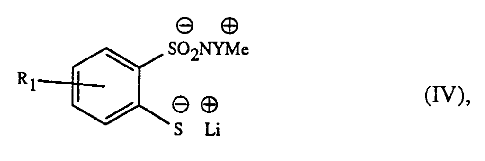 worin Me, R₁ und Y die oben angegebene Bedeutung haben,
überführt, diese mit
d) einem 2-Halogenfluorethan der Formel V
X-CH₂CHF-Z (V)
worin X für Chlor oder Brom steht und Z die unter Formel I angegebene Bedeutung hat, zu einem Phenylsulfonamid der Formel VI 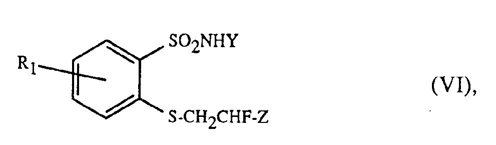 worin Z und R₁ die unter Formel I angegebene Bedeutung und Y die unter Formel II angegebene Bedeutung haben, umsetzt und
B) anschliessend die Gruppe Y abspaltet.

2. Verfahren nach Anspruch 1, dadurch gekennzeichnet, dass R₁ für Wasserstoff steht.

3. Verfahren nach Anspruch 1, dadurch gekennzeichnet, dass Z für Wasserstoff steht.

4. Verfahren nach Anspruch 1, dadurch gekennzeichnet, dass B für OH oder OR₅ steht.

5. Verfahren nach Anspruch 1, dadurch gekennzeichnet, dass man als starke Base der Formel IX Kaliumhydroxid oder Natriummethylat verwendet.

6. Verfahren nach Anspruch 1, dadurch gekennzeichnet, dass X für Brom steht.

7. Verfahren nach Anspruch 1, dadurch gekennzeichnet, dass Y für tert.-Butyl steht.

8. Verfahren nach Anspruch 1, dadurch gekennzeichnet, dass man für die Abspaltung der Gruppe Y Salzsäure verwendet.

9. Verfahren nach Anspruch 1, dadurch gekennzeichnet, dass man
A) N-tert.-Butyl-phenylsulfonamid nacheinander ohne Isolierung von Zwischenprodukten mit
a) Natriummethylat oder Kaliumhydroxid bei einer Temperatur von +60 bis +150°C in die Verbindung der Formel IIIa 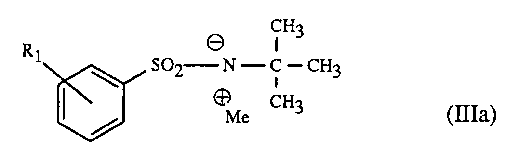 worin Me für Natrium oder Kalium steht und R₁ die unter Formel I in Anspruch 1 angegebene Bedeutung hat, überführt, diese
b) mit einem Äquivalent n-Butyllithium bei einer Temperatur von -20 bis +30°C und
c) Schwefel bei einer Temperatur von -20 bis +30°C
in die Verbindung der Formel IVa 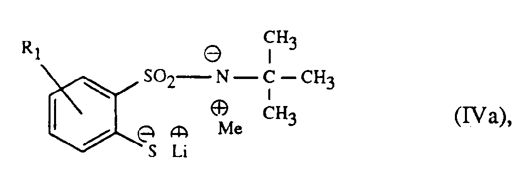 worin Me für Natrium oder Kalium steht und R₁ die unter Formel I in Anspruch 1 angegebene Bedeutung hat, überführt, diese mit
d) einem 2-Halogenfluorethan der Formel V bei einer Temperatur von +20 bis +60°C und einem Druck von 1 bis 3 bar
zu einem 2-(2-Halogenethylthio)-N-tert.-butyl-phenylsulfonamid der Formel VIa 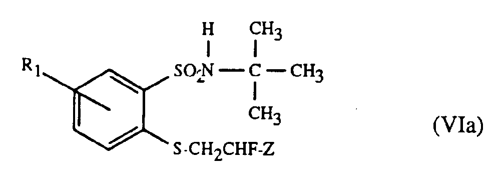 worin Z und R₁ die unter Formel I angegebene Bedeutung haben,
umsetzt, und
B) anschliessend die tert.-Butylgruppe bei einer Temperatur von -20 bis +30°C in Gegenwart von Salzsäure abspaltet.

10. Verfahren nach Anspruch 9, dadurch gekennzeichnet, dass R₁ für Wasserstoff steht.

11. Verfahren nach Anspruch 9, dadurch gekennzeichnet, dass Z für Wasserstoff steht.

## Claims (Claims for the following Contracting State(s): AT, BE, CH, DE, DK, FR, GB, GR, IT, LI, LU, NL, SE)

1. A process for the preparation of a 2-(2-haloethylthio)-phenylsulfonamide of formula I 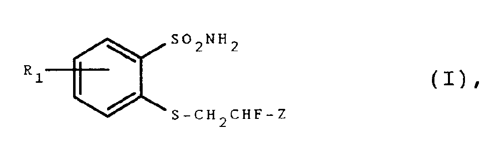 wherein
R₁ is hydrogen, C₁-C₅alkyl or C₂-C₅alkenyl; and
Z is hydrogen, fluorine or chlorine;
which comprises
A) converting a phenylsulfonamide of formula II 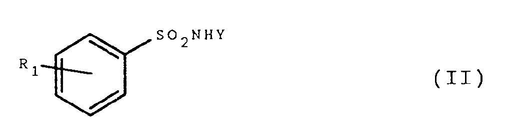 wherein
R₁ is as defined under formula I and
Y is a tertiary alkyl group having from 4 to 8 carbon atoms, benzyl, diphenylmethyl, triphenylmethyl or a silyl group of formula VII
-Si(R₂R₃R₄) (VII),
wherein each of R₂, R₃ and R₄, independently of the others, is C₁-C₆alkyl or phenyl, in succession and without isolating intermediates, with
a) a strong base of formula IX
MeB (IX),
wherein Me is sodium or potassium, B is hydrogen, OH, NH₂ or OR₅ and
R₅ is C₁-C₅alkyl, into the compound of formula III 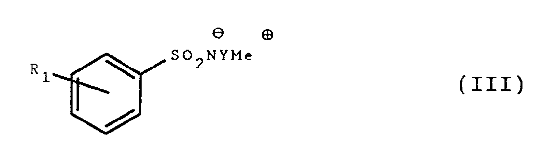 wherein Me, R₁ and Y are as defined above,
converting that compound with
b) one equivalent of n-butyllithium and
c) sulfur
into the compound of formula IV 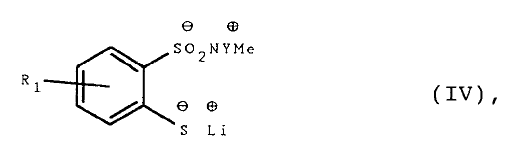 wherein Me, R₁ and Y are as defined above,
reacting that compound with
d) a 2-halofluoroethane of formula V
X-CH₂CHF-Z (V)
wherein X is chlorine or bromine and Z is as defined under formula I, to form a phenylsulfonamide of formula VI 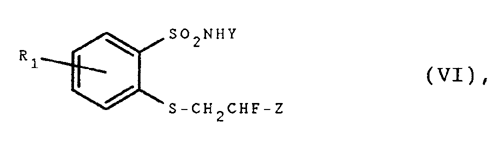 wherein Z and R₁ are as defined under formula I and Y is as defined under formula II and
B) then removing the group Y.

2. A process according to claim 1, wherein R₁ is hydrogen.

3. A process according to claim 1, wherein Z is hydrogen.

4. A process according to claim 1, wherein B is OH or OR₅.

5. A process according to claim 1, wherein potassium hydroxide or sodium methoxide is used as the strong base of formula IX.

6. A process according to claim 1, wherein X is bromine.

7. A process according to claim 1, wherein Y is tert-butyl.

8. A process according to claim 1, wherein hydrochloric acid is used for the removal of the group Y.

9. A process according to claim 1, which comprises
A) converting N-tert-butylphenylsulfonamide, in succession and without isolating intermediates, with
a) sodium methoxide or potassium hydroxide at a temperature of from +60 to +150°C into the compound of formula IIIa 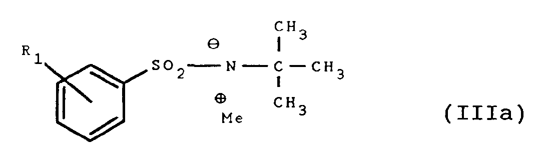 wherein Me is sodium or potassium and R₁ is as defined under formula I in claim 1, converting that compound
b) with one equivalent of n-butyllithium at a temperature of from -20 to +30°C and
c) sulfur at a temperature of from -20 to +30°C
into the compound of formula IVa 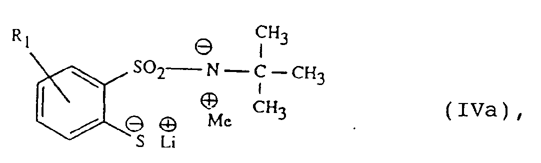 wherein Me is sodium or potassium and R₁ is as defined under formula I in claim 1, reacting that compound with
d) a 2-halofluoroethane of formula V at a temperature of from +20 to +60°C and a pressure of from 1 to 3 bar
to form a 2-(2-haloethylthio)-N-tert-butylphenylsulfonamide of formula VIa 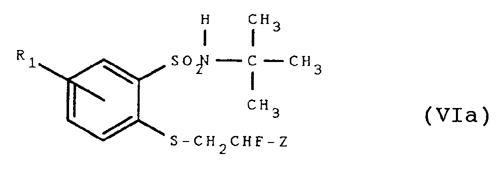 wherein Z and R₁ are as defined under formula I, and
B) then removing the tert-butyl group at a temperature of from -20 to +30°C in the presence of hydrochloric acid.

10. A process according to claim 9, wherein R₁ is hydrogen.

11. A process according to claim 9, wherein Z is hydrogen.

12. A compound of formula VI 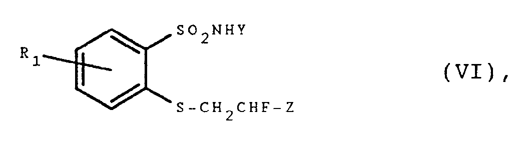 wherein R₁ is as defined under formula I in claim 1, and
Y is a tertiary alkyl group having from 4 to 8 carbon atoms, benzyl, diphenylmethyl, triphenylmethyl or a silyl group of formula VII
-Si(R₂R₃R₄) (VII),
wherein each of R₂, R₃ and R₄, independently of the others, is C₁-C₆alkyl or phenyl.

13. A compound of formula VI according to claim 12, wherein R₁ is hydrogen.

14. A compound of formula VI according to claim 12, wherein Z is hydrogen.

15. A compound of formula VIa 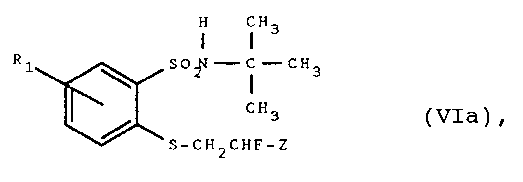 wherein R₁ and Z are as defined under formula I in claim 1.

16. A compound of formula VIa according to claim 15, wherein R₁ is hydrogen.

17. A compound of formula VIa according to claim 15, wherein Z is hydrogen.

## Claims (Claims for the following Contracting State(s): ES)

1. A process for the preparation of a 2-(2-haloethylthio)-phenylsulfonamide of formula I 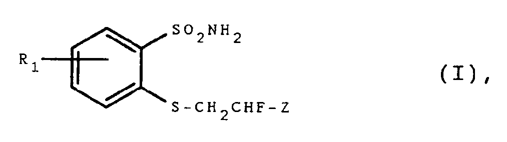 wherein
R₁ is hydrogen, C₁-C₅alkyl or C₂-C₅alkenyl; and
Z is hydrogen, fluorine or chlorine;
which comprises
A) converting a phenylsulfonamide of formula II 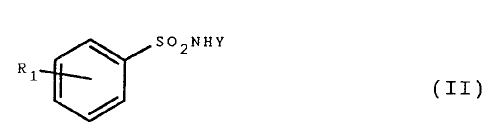 wherein
R₁ is as defined under formula I and
Y is a tertiary alkyl group having from 4 to 8 carbon atoms, benzyl, diphenylmethyl, triphenylmethyl or a silyl group of formula VII
-Si(R₂R₃R₄) (VII),
wherein each of R₂, R₃ and R₄, independently of the others, is C₁-C₆alkyl or phenyl, in succession and without isolating intermediates, with
a) a strong base of formula IX
MeB (IX),
wherein Me is sodium or potassium, B is hydrogen, OH, NH₂ or OR₅ and
R₅ is C₁-C₅alkyl, into the compound of formula III 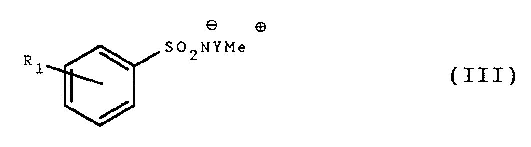 wherein Me, R₁ and Y are as defined above,
converting that compound with
b) one equivalent of n-butyllithium and
c) sulfur
into the compound of formula IV 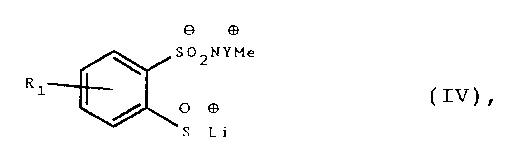 wherein Me, R₁ and Y are as defined above,
reacting that compound with
d) a 2-halofluoroethane of formula V
X-CH₂CHF-Z (V)
wherein X is chlorine or bromine and Z is as defined under formula I, to form a phenylsulfonamide of formula VI 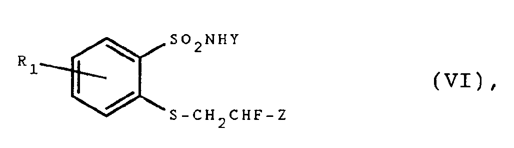 wherein Z and R₁ are as defined under formula I and Y is as defined under formula II and
B) then removing the group Y.

2. A process according to claim 1, wherein R₁ is hydrogen.

3. A process according to claim 1, wherein Z is hydrogen.

4. A process according to claim 1, wherein B is OH or OR₅.

5. A process according to claim 1, wherein potassium hydroxide or sodium methoxide is used as the strong base of formula IX.

6. A process according to claim 1, wherein X is bromine.

7. A process according to claim 1, wherein Y is tert-butyl.

8. A process according to claim 1, wherein hydrochloric acid is used for the removal of the group Y.

9. A process according to claim 1, which comprises
A) converting N-tert-butylphenylsulfonamide, in succession and without isolating intermediates, with
a) sodium methoxide or potassium hydroxide at a temperature of from +60 to +150°C into the compound of formula IIIa 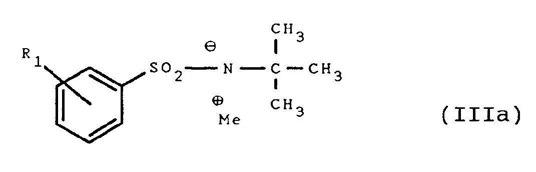 wherein Me is sodium or potassium and R₁ is as defined under formula I in claim 1, converting that compound
b) with one equivalent of n-butyllithium at a temperature of from -20 to +30°C and
c) sulfur at a temperature of from -20 to +30°C
into the compound of formula IVa 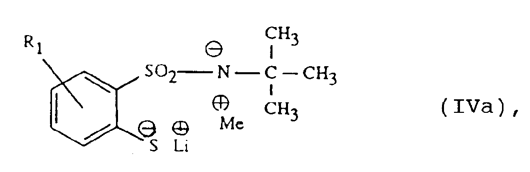 wherein Me is sodium or potassium and R₁ is as defined under formula I in claim 1, reacting that compound with
d) a 2-halofluoroethane of formula V at a temperature of from +20 to +60°C and a pressure of from 1 to 3 bar
to form a 2-(2-haloethylthio)-N-tert-butylphenylsulfonamide of formula VIa 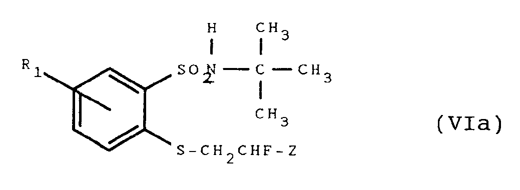 wherein Z and R₁ are as defined under formula I, and
B) then removing the tert-butyl group at a temperature of from -20 to +30°C in the presence of hydrochloric acid.

10. A process according to claim 9, wherein R₁ is hydrogen.

11. A process according to claim 9, wherein Z is hydrogen.

## Revendications (Revendications pour l'(les) Etat(s) contractant(s) suivant(s): AT, BE, CH, DE, DK, FR, GB, GR, IT, LI, LU, NL, SE)

1. Procédé de préparation des 2-(2-halogénoéthylthio)-phénylsulfonamides de formule I 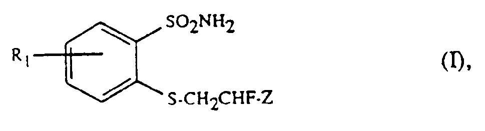 dans laquelle
R₁ représente l'hydrogène, un groupe alkyle en C1-C5 ou alcényle en C2-C5; et
Z représente l'hydrogène, le fluor ou le chlore,
caractérisé en ce que
A) on convertit un phénylsulfonamide de formule II 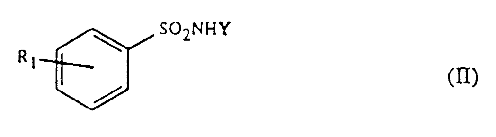 dans laquelle
R₁ a les significations indiquées en référence à la formule I et
Y représente un groupe tert-alkyle en C4-C8, benzyle, diphénylméthyle, triphénylméthyle, ou un groupe silyle de formule VII
-Si(R₂R₃R₄) (VII),
dans laquelle R₂, R₃ et R₄ représentent chacun, indépendamment les uns des autres, un groupe alkyle en C1-C6 ou phényle,
successivement, sans isolement des produits intermédiaires, à l'aide de
a) une base forte de formule IX
MeB (IX)
dans laquelle Me représente le sodium ou le potassium, B représente l'hydrogène, un groupe OH, NH₂ ou OR₅ et R₅ représente un groupe alkyle en C1-C5, en le composé de formule III 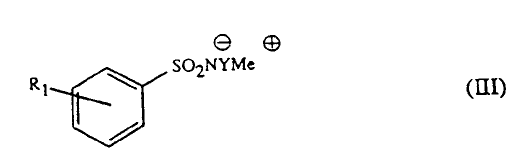 dans laquelle Me, R₁ et Y ont les significations indiquées ci-dessus,
qu'on convertit lui-même à l'aide de
b) un équivalent de n-butyl-lithium et
c) le soufre
en le composé de formule IV 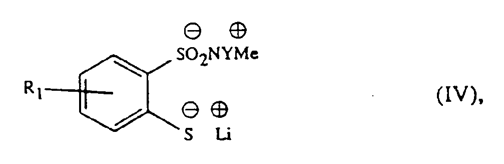 dans laquelle Me, R₁ et Y ont les significations indiquées ci-dessus,
lequel est mis à réagir avec
d) un 2-halogénofluoréthane de formule V
X-CH₂CHF-Z (V)
dans laquelle X représente le chlore ou le brome et Z a les significations indiquées en référence à la formule I, ce qui donne un phénylsulfonamide de formule VI 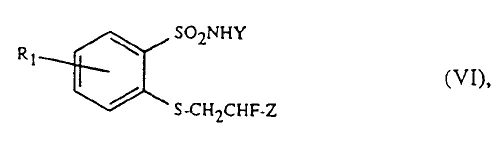 dans laquelle Z et R₁ ont les significations indiquées en référence à la formule I et Y les significations indiquées en référence à la formule II,
B) d'où l'on élimine le groupe Y.

2. Procédé selon revendication 1, caractérisé en ce que R₁ représente l'hydrogène.

3. Procédé selon revendication 1, caractérisé en ce que Z représente l'hydrogène.

4. Procédé selon revendication 1, caractérisé en ce que B représente OH ou OR₅.

5. Procédé selon revendication 1, caractérisé en ce que la base forte de formule IX qui est utilisée est l'hydroxyde de potassium ou le méthylate de sodium.

6. Procédé selon revendication 1, caractérisé en ce que X représente le brome.

7. Procédé selon revendication 1, caractérisé en ce que Y représente un groupe tert-butyle.

8. Procédé selon revendication 1, caractérisé en ce que, pour la scission du groupe Y, on utilise de l'acide chlorhydrique.

9. Procédé selon revendication 1, caractérisé en ce que
A) on convertit le N-tert-butyl-phénylsulfonamide, successivement, sans isolement des produits intermédiaires, à l'aide de
a) le méthylate de sodium ou l'hydroxyde de potassium, à une température de +60 à +150°C, en le composé de formule IIIa 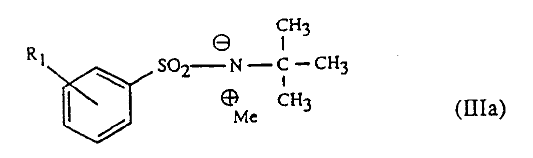 dans laquelle Me représente le sodium ou le potassium et R₁ a les significations indiquées en référence à la formule I dans la revendication 1, qu'on convertit lui-même
b) à l'aide d'un équivalent de n-butyl-lithium, à une température de -20 à +30°C et
c) à l'aide du soufre, à une température de -20 à +30°C, en le composé de formule IVa 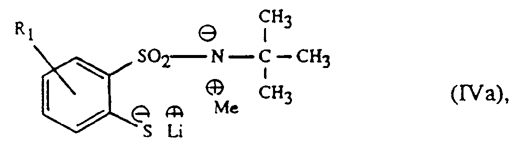 dans laquelle Me représente le sodium ou le potassium et R₁ a les significations indiquées en référence à la formule I dans la revendication 1, qu'on fait réagir
d) avec un 2-halogénofluoréthane de formule V à une température de +60°C sous une pression de 1 à 3 bar,
la réaction donnant un 2-(2-halogénoéthylthio)-N-tert-butyl-phénylsulfonamide de formule VIa 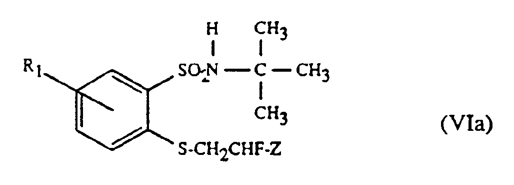 dans laquelle Z et R₁ ont les significations indiquées en référence à la formule I, après quoi
B) on élimine le groupe tert-butyle à une température de -20 à +30°C en présence d'acide chlorhydrique.

10. Procédé selon revendication 9, caractérisé en ce que R₁ représente l'hydrogène.

11. Procédé selon revendication 9, caractérisé en ce que Z représente l'hydrogène.

12. Composés de formule VI 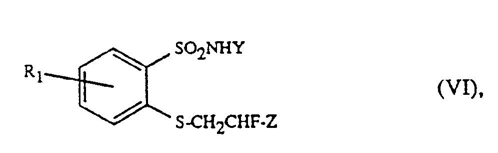 dans laquelle R₁ a les significations indiquées en référence à la formule I dans la revendication 1 et
Y représente un groupe tert-alkyle en C4-C8, benzyle, diphényle, triphénylméthyle ou un groupe silyle de formule VII
-Si(R₂R₃R₄) (VII),
dans laquelle R₂, R₃ et R₄ représentent chacun, indépendamment les uns des autres, un groupe alkyle en C1-C6 ou phényle.

13. Composés de formule VI selon revendication 12, caractérisés en ce que R₁ représente l'hydrogène.

14. Composés de formule VI selon revendication 12, caractérisés en ce que Z représente l'hydrogène.

15. Composés de formule VIa 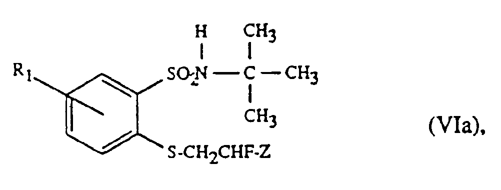 dans laquelle R₁ et Z ont les significations indiquées en référence à la formule I dans la revendication 1.

16. Composés de formule VIa de la revendication 15, caractérisés en ce que R₁ représente l'hydrogène.

17. Composés de formule VIa de la revendication 15, caractérisés en ce que Z représente l'hydrogène.

## Revendications (Revendications pour l'(les) Etat(s) contractant(s) suivant(s): ES)

1. Procédé de préparation des 2-(2-halogénoéthylthio)-phénylsulfonamides de formule I 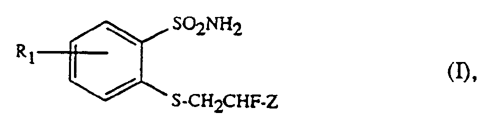 dans laquelle
R₁ représente l'hydrogène, un groupe alkyle en C1-C5 ou alcényle en C2-C5; et
Z représente l'hydrogène, le fluor ou le chlore,
caractérisé en ce que
A) on convertit un phénylsulfonamide de formule II 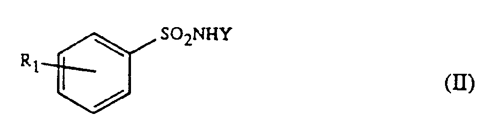 dans laquelle
R₁ a les significations indiquées en référence à la formule I et
Y représente un groupe tert-alkyle en C4-C8, benzyle, diphénylméthyle, triphénylméthyle ou un groupe silyle de formule VII
-Si(R₂R₃R₄) (VII),
dans laquelle R₂, R₃ et R₄ représentent chacun, indépendamment les uns des autres, un groupe alkyle en C1-C6 ou phényle,
successivement, sans isolement des produits intermédiaires, à l'aide de
a) une base forte de formule IX
MeB (IX)
dans laquelle Me représente le sodium ou le potassium,
B représente l'hydrogène, un groupe OH, NH₂ ou OR₅ et R₅ représente un groupe alkyle en C1-C5, en le composé de formule III 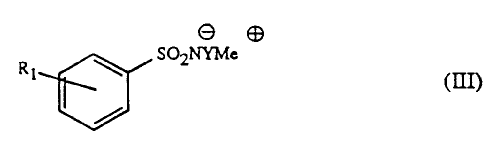 dans laquelle Me, R₁ et Y ont les significations indiquées ci-dessus,
qu'on convertit lui-même, à l'aide de
b) un équivalent de n-butyl-lithium et
c) le soufre en le composé de formule IV 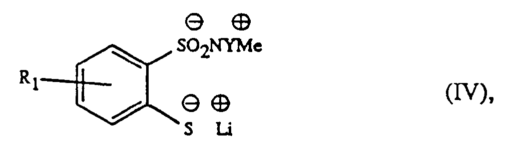 dans laquelle Me, R₁ et Y ont les significations indiquées ci-dessus,
lequel est ensuite mis à réagir avec
d) un 2-halogénofluoréthane de formule V
X-CH₂CHF-Z (V)
dans laquelle X représente le chlore ou le brome et Z a les significations indiquées en référence à la formule I, ce qui donne un phénylsulfonamide de formule VI 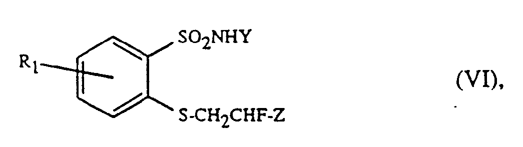 dans laquelle Z et R₁ ont les significations indiquées en référence à la formule I et Y a les significations indiquées en référence à la formule II et
B) on élimine ensuite le groupe Y.

2. Procédé selon revendication 1, caractérisé en ce que R₁ représente l'hydrogène.

3. Procédé selon revendication 1, caractérisé en ce que Z représente l'hydrogène.

4. Procédé selon revendication 1, caractérisé en ce que B représente OH ou OR₅.

5. Procédé selon revendication 1, caractérisé en ce que la base forte de formule IX qui est utilisée est l'hydroxyde de potassium ou le méthylate de sodium.

6. Procédé selon revendication 1, caractérisé en ce que X représente le brome.

7. Procédé selon revendication 1, caractérisé en ce que Y représente le groupe tert-butyle.

8. Procédé selon revendication 1, caractérisé en ce que, pour la scission du groupe Y, on utilise de l'acide chlorhydrique.

9. Procédé selon revendication 1, caractérisé en ce que
A) on convertit le N-tert-butyl-phénylsulfonamide, successivement, sans isolement des produits intermédiaires, à l'aide de
a) le méthylate de sodium ou l'hydroxyde de potassium, à une température de +60 à +150°C, en le composé de formule IIIa 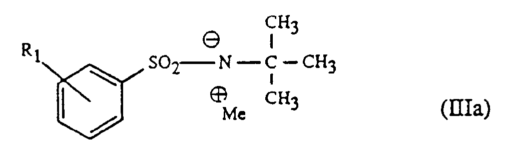 dans laquelle Me représente le sodium ou le potassium et R₁ a les significations indiquées en référence à la formule I dans la revendication 1, lequel est ensuite converti
b) à l'aide d'un équivalent de n-butyl-lithium à une température de -20 à +30°C et
c) à l'aide du soufre à une température de -20 à +30°C en le composé de formule IVa 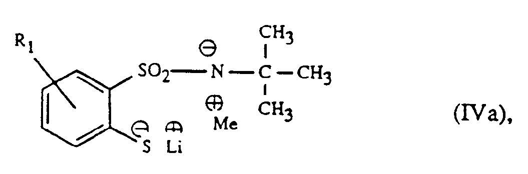 dans laquelle Me représente le sodium ou le potassium et R₁ a les significations indiquées en référence à la formule I dans la revendication 1, qu'on fait ensuite réagir
d) avec un 2-halogénofluoréthane de formule V, à une température de +20 à +60°C et sous une pression de 1 à 3 bar, la réaction donnant un 2-(2-halogénoéthylthio)-N-tert-butyl-phénylsulfonamide de formule VIa 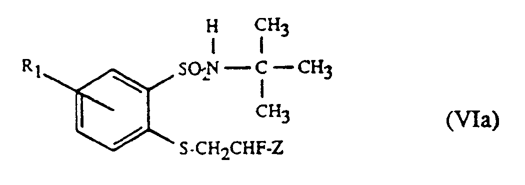 dans laquelle Z et R₁ ont les significations indiquées en référence à la formule I,
B) d'où l'on élimine ensuite le groupe tert-butyle à une température de -20 à +30°C en présence d'acide chlorhydrique.

10. Procédé selon revendication 9, caractérisé en ce que R₁ représente l'hydrogène.

11. Procédé selon revendication 9, caractérisé en ce que Z représente l'hydrogène.
